# EUROPEAN PATENT SPECIFICATION

(11) **EP 2 798 585 B1**
(45) Date of publication and mention of the grant of the patent: **07.02.2018**
(21) Application number: 12818717.6
(22) Date of filing: 18.12.2012
(51) Int. Cl.: G06K 19/077, G01K 3/04, G01V 15/00

(54) **RFID TRANSPONDER COMPRISING SENSOR ELEMENT**
RFID-TRANSPONDER MIT SENSOR
TRANSPONDEUR RFID DOTÉ D'UN DÉTECTEUR

(30) Priority: 27.12.2011 US 201161580379 P; 14.09.2012 US 201213616373
(43) Date of publication of application: 05.11.2014
(73) Proprietor: The Gillette Company LLC, Boston, MA 02127 (US)
(72) Inventor: AMANN, Mathias, 64295 Darmstact (DE); STRIEMER, Grant, Edward, Hamilton, Ohio 45013 (US); SHERMAN, Faiz, Feisal, Mason, Ohio 45040 (US); JOYCE, Jonathan, Livingston, Independence, Kentucky 41051 (US); BOURILKOV, Jordan, Torodov, Bethany, Connecticut 06524 (US); MORROW, Mark, Wayne, Milford, Ohio 45150 (US); FRANKE, Michael, 64291 Darmstadt (DE); SPECHT, Steven, Jeffrey, Brookfield, Connecticut 06804 (US)
(74) Representative: Zetterer, Gerd
(86) International application number: PCT/US2012/070274
(87) International publication number: WO 2013/101539

(56) References cited:
- DE-A1- 10 012 204
- DE-A1-102004 056 379
- DE-B3- 10 314 758
- US-A1- 2004 008 112
- US-A1- 2007 152 829
- US-A1- 2008 042 830
- US-A1- 2009 045 918
- US-A1- 2009 195 356
- US-B1- 6 342 830

## Description

### FIELD OF THE INVENTION

The invention relates to systems and methods for determining information about a product according to the preamble of the independent claims. The invention relates particularly to the remote interrogation of product information and the subsequent use of the acquired information.

### BACKGROUND OF THE INVENTION

Consumable goods having a useful life defined in terms of the consumption of the goods are well known. The useful life may be viewed as one or more events associated with the consumption of at least a portion of the useful quantity of the goods. In formation associated with the environment of use of the goods and/or the quantity of goods used and remaining available may exist but may also be generally inaccessible to the typical consumer of the goods. What is needed is a system and method for extracting product relevant information in a manner which makes the information readily accessible and usable by the consumer.

DE 103 14 758 B3 discloses a temperature monitoring device for food or medicaments during storage and transport. It uses a microchip with a temperature sensor. The sensor consists of a constant current source and a temperature dependent resistor. A voltage controlled oscillator connected to a counter serves as a measuring device and provides an integrated value of the temperature to the device memory. In addition, a comparator detects whether the measured temperature exceeds a given threshold, and alters a bit in the device's excess temperature register. These values can be read by an interrogator via a radio connection.

From US 2009/0045918 an electronic label with RFID transponder and e-ink display for monitoring refrigerated products, for example food or medicaments is known. The label comprises a number of layers, including an antenna shielding layer. A temperature sensor detects the ambient temperature. The processor processes the data received from the sensor such that said data are stored in a memory. An alarm signal is supplied to the display, if a predefined temperature range is exceeded.

### SUMMARY OF THE INVENTION

The invention is defined by the independent claims.

Preferred embodiments are set out in the dependent claims.

### BRIEF DESCRIPTION OF THE DRAWINGS

Further features and benefits of the present invention will become more readily apparent through consideration of the drawings.
Figure 1 shows a schematic representation of one embodiment of the invention.
Figure 2 show s a schematic representation of one embodiment of the invention.
Figure 3 shows a schematic representation of one embodiment of the invention.

### DETAILED DESCRIPTION OF THE INVENTION

In one aspect, a sensor system comprising a tag. The tag may comprise one or more layers of conductive inks and non-conductive inks printed upon a substrate. Exemplary substrate materials include: polymer films, paper, high permittivity dielectric materials, and FR-4 material. Multiple layer structures may further comprise partial layers of non-conducting material separating at least portions of the conductive layers. Exemplary conductive layers include copper and silver inks.

The tag comprises at least one sensor, a radio-frequency chip, and a first antenna disposed as a circuit upon a card, coin, or inlay. The chip may be active or passive. Exemplary chip/first antenna combinations include model numbers: RI-I03-112A-03 (13.56 MHz), andRI-INL-R9QM (134.2 kHz), or model TRF7970A, each available from Texas Instruments, Dallas, TX. The antenna may be in the physical form of a coil or a dipole, or a conductive component of a product or package in electrical communication with the remainder of the tag.

The sensor may be selected according to the nature of the environmental factor of interest. Typical sensor types include chemical sensors, electrical sensors, biological sensors, mechanical sensors, and physical sensors. The sensor of the tag may also comprise multiple sensor of a single type or a combination of sensors of differing types.

Exemplary chemical sensors include: model TGS 813 gas sensor for Propane, Butane, Methane, Alcohol, H2 made by Figaro Engineering Inc., and available from Conrad Electronics, of Wernberg-Koblitz, Germany . Exemplary electrical sensors include: model AH1751-PG-B-A Hall effect sensor to measure magnetic field made by Zetex Semiconductors, and available from Allied Electronics, of Fort Worth, TX.

Exemplary mechanical sensors include: model L3G3200D 3-axis gyro based on MEMS made by ST Microelectronics, and model N11MA512023 strain gauge sensor made by Allied Electronic, Forth Worth, TX.

Exemplary physical sensors include: model- VCNL4020 light sensor made by Vishay, and model LPS331AP pressure sensor based on MEMS made by ST Microelectronics.

Exemplary biological sensors can be tailored to detect various biological molecules such as diseases, ions, bio markers, antibodies, DNA, various proteins, or metabolic markers, etc. The method of detection fundamentally can be potentiometric or amperometric in nature. Analyzed material samples may be collected from an organism or from the environment and include: blood, epithelial cells, mucous, saliva, feces, hair, urine, air, water and other environmental materials. The electrode configuration can be two (working and counter) or three (working, counter and reference) where the electrodes can be made of a number of substrates such as gold, silver, platinum, carbon, etc. These electrodes may be purchased from Conductive Technologies, Inc., of York, PA, or made (Sensors and Actuators B, 114, (2006) 357-363).

A potentiometric bio sensor that detects antibodies may take advantage of a conventional ELISA sandwich assay. The previously mentioned electrodes are coated with a conductive polymer such as poly(pryyole) to prepare a substrate suitable for bio attachment while maintaining conductivity to the electrode substrate. The materials for the assay may be purchased from Sigma-Aldrich, of St. Louis, MO.: capture antibody - anti-mouse IgG (Fc specific) F(ab')₂ fragment antibody produced in goat (M0284), antibody - mouse serum (M5905), analyte competitor - anti-mouse IgG (γ-chain specific) peroxidase antibody produced in goat (A3673) and albumin from bovine serum is used during the process to prevent non-specific binding (A7906).

The ELISA sandwich structure described from the bottom up on the working electrode, is as follows: capture antibody adsorption to the poly(pyrrole) substrate, analyte, analyte competitor with HRP conjugate, and working solution enzyme to catalyze the HRP to generate hydrogen peroxide. This reaction at the working electrode generates a potential with a resolution of micro volts.

The needed power supply for the sensors may be provided by the harvested energy of the RFID circuit because the needed current is in the micro ampere range. The harvested power may be stored with an element such as a capacitor for use by the sensor at a later time.

The tag may be configured such that the output of the sensor alters the value of one or more bits of the word stored in the memory of the tag's chip. In one embodiment, any non-zero sensor output may alter a designated bit's value either from one to zero or from zero to one.

Alternatively, the tag's circuit may provide a bias against which the sensor output is compared. In this embodiment, only sensor outputs above the bias threshold, or between a lower and upper set of thresholds may alter the bit's value.

The tag may comprise more than a single sensor. In one configuration of a multi sensor tag, each sensor's output may be used to alter the value of its own respective bit. In an alternative configuration, the set of sensors may be polled when the tag is powered such that a single particular bit of the tag's memory is stepped through a series of values depending upon the output of each polled sensor. As noted before, the tag may be designed such that any non-zero sensor output will alter the value of the associated bit, or such that only values above a lower threshold, or between an upper and lower threshold will alter the respective bit value.

The tag may be read using an radio frequency protocol such as the Near Field Communications (NFC) protocol. When the tag is interrogated, or read, the tag circuit is powered, the sensor output alters the memory of the tag. The memory of the tag is then read by an interrogator. The acquires a digital value of the memory word indicative of the sensed state of the environment of the tag. The relevant communications frequency range of the tag may be HF, UHF or other appropriately selected frequency ranges as determined by the specific need of the tag in terms of the intended environment and uses of the tag.

The sensor system may further comprise an interrogator. The interrogator comprising a power source and a second antenna adapted to generate electromagnetic radiation comprising a resonant frequency of the first antenna, and a receiver adapted to detect electromagnetic radiation and de-modulate the detected radiation extracting embedded data from the detected radiation. The Bluetooth^{tm} RFID Reader, model number 223012, available GAO RFID, of Toronto Canada, exemplifies one form of interrogator. The model 223012 interrogator has the capacity to interrogate the radio frequency tag and to determine the state of the memory of the tag and thus extract information associated with the output of the sensor or sensors relating to the environment of the tag. The 223012 further comprises a secondary network communications link utilizing the Bluetooth^{tm} communications protocol for transmitting the information extracted from the tag to a secondary device or secondary interrogator, such as a Bluetooth^{tm} enabled computer or smart phone. The secondary interrogator may further analyze the information relating to the state of the tag and/or the tags environment and provide an output associated with a particular tag and/or tag environment state. The interrogator may further comprise a display element such as an LCD or LED screen for displaying an output associated with the analyzed tag information. The interrogator may further comprise one or more sensors for ascertaining information associated with the environment of the interrogator. The sensors may include: temperature, humidity, acceleration sensors. The interrogator may further comprise one or more cameras enabling the capture of images associated with a product, the tag or the environment. The interrogator may comprise a Global Positioning capability enabling the interrogator to ascertain and share information relating to the geographic location of the interrogator.

In one aspect, the Smartphone may serve as the only interrogator. In this aspect the smart phone may interrogate the tag thereby ascertaining the information from the memory of the tag. The interrogator may analyze or otherwise interpret the information and may create an output. The output may be provided to a system user via an audio output, visual output, haptic output or combinations thereof. The interrogator may utilize inputs from sensors or systems of the smart phone, including information and analysis available from a networked resource such as cloud computing resources, in addition to the tag information in creating the output. Exemplary smart phones suitably configured to perform as a system interrogator include: the Acer^{tm} E320 Liquid Express, the Blackberry^{tm}, Bold^{tm} 970, available from Research In Motion of ; the Casio IT-800; the Google Nexus 7^{tm}, available from Google, Inc. Mountain View Ca.; the HTC Desire C^{tm}, available from HTC of; the LG Optimus Elite; the Motorola Droid^{tm} Razr^{tm}, available from Motorola; the Nokia 700; the Panasonic BizPad^{tm}; and the Samsung Galaxy S Advance^{tm}.

In one aspect, the sensor system may include a product. The term "product(s)" is used in the broadest sense and refers to any product, product group, services, communications, entertainment, environments, organizations, systems, tools, and the like. For example, an example of a product group is personal and household products, such as used by a person, family or household. Examples of a representative, and non-limiting list of product categories within the personal and household product group includes antiperspirants, baby care, colognes, commercial products (including wholesale, industrial, and commercial market analogs to consumer-oriented consumer products), cosmetics, deodorants, dish care, feminine protection, hair care, hair color, health care, household cleaners, laundry, oral care, paper products, personal cleansing, disposable absorbent articles, pet health and nutrition, prescription drugs, prestige fragrances, skin care, foods, snacks and beverages, special fabric care, shaving and other hair growth management products, small appliances, devices and batteries, services such as haircutting, beauty treatment, spa treatment, medical, dental, vision services, entertainment venues such as theaters, stadiums, as well as entertainment services such as film or movie shows, plays and sporting events A variety of product forms may fall within each of these product categories.

Exemplary product forms and brands are described on The Procter & Gamble Company's website www.pg.com, and the linked sites found thereon. It is to be understood that consumer products that are part of product categories other than those listed above are also contemplated by the present invention, and that alternative product forms and brands other than those disclosed on the above-identified website are also encompassed by the present invention.

Exemplary products within the laundry category include detergents (including powder, liquid, tablet, and other forms), bleach, conditioners, softeners, anti-static products, and refreshers (including liquid refreshers and dryer sheets). Exemplary products within the oral care category include dentifrice, floss, toothbrushes (including manual and powered forms), mouth rinses, gum care products, tooth whitening products, and other tooth care products. Exemplary feminine protection products include pads, tampons, interlabial products, and pantiliners. Exemplary baby care products include diapers, wipes, baby bibs, baby change and bed mats, and foaming bathroom hand soap.

Exemplary health care products include laxatives, fiber supplements, oral and topical analgesics, gastro-intestinal treatment products, respiratory and cough/cold products, heat delivery products, and water purification products. Exemplary paper products include toilet tissues, paper towels, and facial tissues. Exemplary hair care products include shampoos, conditioners (including rinse-off and leave-in forms), and styling aids. Exemplary household care products include sweeper products, floor cleaning products, wood floor cleaners, antibacterial floor cleaners, fabric and air refreshers, and vehicle washing products. Skin care products include, but are not limited to, body washes, facial cleansers, hand lotions, moisturizers, conditioners, astringents, exfoliation products, micro-dermabrasion and peel products, skin rejuvenation products, anti-aging products, masks, UV protection products, and skin care puffs, wipes, discs, clothes, sheets, implements and devices (with or without skin care compositions).

In one embodiment, the product may comprise a test unit for the evaluation of other products such as portable power sources. In this embodiment, the product may comprise all the described elements including the chip, antenna, and shielding. In using this embodiment, the user will place the power source of interest in the product and engage the tag of the unit using the interrogator. The interrogator will power the tag, read and analyze the memory value, and generate an output. The analysis will evaluate the read value in terms of the open circuit voltage of the power source to which it is analogous. The output may be in the form of a percentage of power remaining, a color coded output associated with the power remaining, a simple textual output, good or bad, etc., or combinations of these. The output may be further transmitted using a network communications link and/or displayed using the display element of the interrogator.

Other product groups include but are not limited to: sports equipment, entertainment (books, movies, music, etc), vision, and in-home-consumed medical and first aid, among others.

The tag may be attached to the packaging of the product such as the primary packaging of a liquid product, or a granular product. The tag may be immersed in or float upon the surface of a packaged liquid or granular product. The tag may be incorporated within the product such as within a disposable absorbent article such as within a diaper for the purpose of detecting an insult to the absorbent core of the diaper. The tag may be disposed upon the surface of the product itself such as upon the surface of a battery for the purpose of sensing information relating to the useful power remaining in the battery.

It is believed that conforming the antenna of the tag to the shape of the outer surface of the product yields a system where communication between the interrogator and the tag may be omnidirectional or achievable at a variety of angles between the interrogator and the tag.

One of the problems associated with creating a communication device for various products is realized when the communication device is utilized on electromagnetically conductive bodies. Free space radio propagation principles do not apply near highly conductive bodies. Additionally, antenna performance is severely degraded when antennas are placed near metals. As such, simply placing an RFID tag on a battery or on an object with a conductive body may not accomplish the desired effect, e.g. power harvesting and/or data transfer. Notably, this problem is not limited to rechargeable / disposable batteries. For example, a can of shaving gel, foam, etc., or a package comprising a metalized film, could experience the same issues because of the conductivity of the container. In general, an RFID tag next to metallic body decreases signal coupling between the reader and the tag by 10x.

One way to prevent the effects arising from metal proximity to the antenna is to prevent the electromagnetic field from entering the metal. For example, separating the antenna and the metal surface by placing a material with suitable electromagnetic properties and dimensions between them may divert the electromagnetic field around the metallic / conductive body of the product. The properties of the diverter material depend on the exact metal used and the RFID frequency. The magnetic diverter effectively isolates the tag from the can. An effective separation may also be achieved with an air filled gap between the materials.

In one aspect, a method of determining product information comprises steps of: providing a product comprising a tag as described above. The tag comprising, at least one sensor adapted to provide an output analogous to a change in an environment of the sensor. The sensor having at least one output terminal. The tag also includes a radio-frequency chip comprising a memory element, input terminal(s) and output terminal(s), the input terminal(s) disposed in electrical communication with the output terminals of the sensor, and a first antenna disposed in electrical communication with the output terminals of the chip.

The method may also include providing an interrogator adapted to detect radiation associated with the data of the tag. The interrogator may be an RF or NFC protocol reader coupled with a Bluetooth^{tm} capability as described above, or a smart phone or other computing device comprising an RF or NFC capable reader.

In one aspect the method may be minimized to providing products including tags and providing software compatible with devices available in the market or in the possession of consumers. A consumer may choose to avail themselves of the application software which will enable their device to functions as the described interrogator.

The interrogator may be used to determine the current state of the tag utilizing an RF communications protocol such as the NFC protocol. The interrogator may interpret the data received from the tag using a software application written for that purpose. In one embodiment, the tag and sensor may be configured to detect an open circuit voltage of a battery cell. The detected open circuit voltage may alter the value of at least one bit of the memory of the tag. The interrogator may then read the value of the memory and correlate that value with an open circuit voltage and a useful battery life remaining. The useful battery life remaining may be displayed to a user of the system via a display element of the interrogator. The output displayed may be in terms of a percentage of life remaining or in more analog terms of red, yellow and green colors signifying little to no useful life, some useful life, or a significant amount of useful life depending upon the algorithm used to set the thresholds for converting the detected open circuit voltage to an analog of battery useful life. In one embodiment, the application may provide steps for the user of the application to input the nature of the load case of the battery. Exemplary load cases include powering: a flash camera, a toy, a flashlight, a remote control unit, a clock, or a radio or other entertainment system.

As an example, the tag may be incorporated as a sensor system for a 1.5 volt alkaline power source. In this example the sensor may be configured to read the open circuit voltage (OCV) in the range between 0.8 volts and 1.65 volts. The biasing circuitry of the tag may be designed to provide for a first bit to change state if the OCV is above 1.35 volts as an indicator that more than 30% of the power remains to be used. This may be tied to an application output of a green color coded state for the power source. A second portion of the tag may provide for a second bit to change state when the OCV is above 1.25 volts as an indication that -30% of the power remains and this may be communicated as a yellow state of power. The tag may be further configured to have a third bit change state when the OCV is less than 1.25 volts and the application may indicate a red power state. Alternatively, the tag bits may be left unchanged either below 1.25 volts or above 1.35 volts to simplify the design of the circuit.

In one aspect, the interrogator may incorporate a secondary network communication module affording the device an ability to send and receive data over a cellular phone or other networks including a local area or wifi networks. In such an aspect, the interrogator may transmit data received from the tag and/or an analysis of the data from the tag. The software application of the interrogator may analyze the data from the tag to determine if replenishment of the product associated with the tag in needed, or to project when such replenishment will be needed in view of usage history of the product established via a series of interrogations of the tag. In this aspect the application may be used to consummate a purchase of addition product via the network. The application may be further utilized to offer the user related products for purchase, or to make offers of other products not directly related to the product.

### Examples:

A tag comprising a capacitive sensor strip coupled with the tag memory may be arrayed within a package, such as a mascara package, such that the sensor output will be analogous to the product quantity remaining within the package. An axis of the sensor may be aligned with the geometry of the package such that the portion of the sensor in contact with the product will change as the amount of product in the container changes. Upon interrogation, the tag sensor will provide an output to the chip analogous to the product quantity remaining. The tag circuit may be biased such that only a sensor above a particular value or within a particular range will alter the value of the bit associate with the output. The tag will provide an indication that there either is or is not a predetermined amount of product remaining based upon the manufacturers knowledge of what quantity of product should be used as the threshold to trigger an output associated with the particular product and/or package. The interrogator will read the stored value. The application software may then analyze the read value and interpret it in terms of the amount of product remaining. The software may then provide an output to the user indicating the amount of product remaining as a quantity or as a percentage of the original amount within the package, or the output may be simply that it is time to replenish the product.. The software may be written to provide a suggestion to the user to replenish the product at a particular quantity or percentage threshold. The software may utilize the network communications capacity of the interrogator to enable the user to connect to a network retail source to purchase replenishment product offered by a network retailer. The software may search the network to identify a set of retailers and may also gather information such as the price and shipping options of the product associated with each retailer. The software may utilize a location provider by the user or derived via the GPS or wifi location capabilities of the interrogator to identify retailers having location near the present location of the user where the product may be directly purchased.

A tag comprising an electrical sensor, such as a sensor for resistance, capacitance, inductance, or combinations thereof, may be provided in contact with a product as part of a product/package combination. The system may be configured to sense changes in the product, such as changes related to shelf stability or the efficacy of the product, and to provide an output associated with such a change in the product.

A tag comprising a temperature sensor may be incorporated within the diaper such that upon interrogation the sensor output to the memory will be analogous to the temperature of the wearer of the diaper which will be stored digitally in the memory of the tag. The application software may read the stored value and interpret it in terms of body temperature. The associated value may be stored by the application software together with other available data such as date, time, location, images of the wearer, and combinations thereof. A collection of data records may be accumulated over time and used as an indicator of the wearer's health and wellness.

A tag comprising a chemical or biosensor coupled to the memory may be incorporated within a patient garment for the purpose of detecting environmental factors associated with the occurrence of pressure ulcers. Other tags having chemical or biological sensors may be used for purposes such as detecting metabolic markers in saliva, detecting alcohol in breath or saliva, detecting malodorous compounds in air samples.

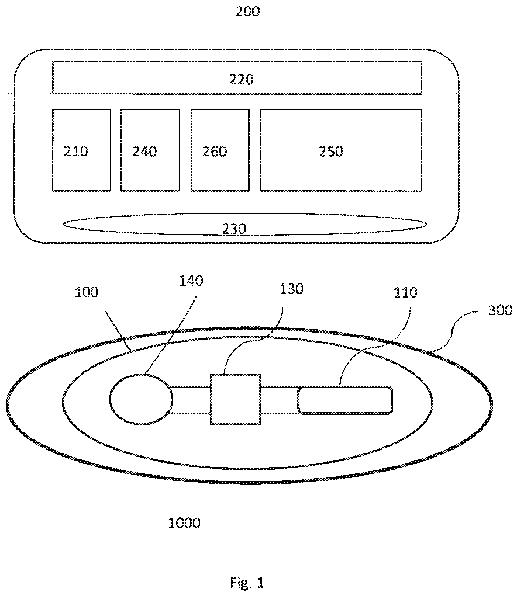

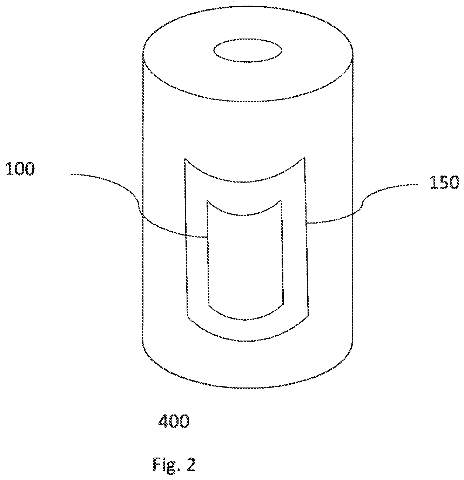

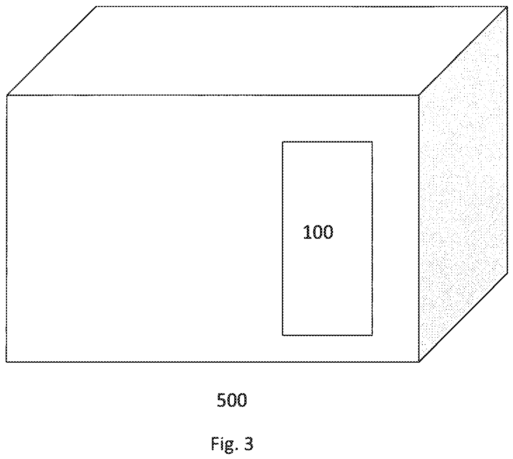

As shown in Figure 1, a system 1000, comprises an absorbent article 300, and an interrogator 200. The absorbent article 300 comprises a tag 100. The tag 100 comprises a sensor 110, a chip 130, and an antenna 140. The interrogator 200 comprises a sensor 210, a power source 220, an antenna 230, an analysis element 240, a display element 250, and a network link 260. As shown in Figure 2, a portable power source 400, comprises a tag 100 and shielding 150. As shown in Figure 3, a package 500, comprises a tag 100.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A sensor system including a tag (100) and a product (300, 400, 500),
the tag (100) including: at least one sensor (110) adapted to transition from a first electrical state to a second electrical state in response to a predetermined change in an environment of the sensor (110), the sensor (110) having at least one output terminal;
a radio-frequency chip (130) including a memory element including electrical storage of a binary coded word including at least one bit, input terminals and output terminals,
the input terminals being disposed in electrical communication with the at least one output terminal of the at least one sensor (110) and with at least one bit of memory;
a first antenna (140) being disposed in electrical communication with the output terminals of the chip;
**characterized in that** the product comprises a battery, wherein the tag and sensor are configured to detect an open circuit voltage of the battery.

2. The sensor system according to claim 1 wherein the at least one output terminal of the sensor (110) is disposed in electrical communication with multiple bits of the memory.

3. The sensor system according to claim 1 further including an interrogator (200) including a power source (220) and a second antenna (230) adapted to generate electromagnetic radiation including a resonant frequency of the first antenna (140), and a receiver adapted to detect electromagnetic radiation and de-modulate the detected radiation extracting embedded data from the detected radiation.

4. The system according to claim 3 wherein the interrogator (200) includes a network communications link (260).

5. The sensor system according to claim 3 wherein the interrogator (200) comprises a sensor (210).

6. The sensor system according to claim 3 wherein the embedded data comprises the value of the at least one bit.

7. The sensor system according to claim 1 further including electrical shielding (150) disposed between the antenna and product.

8. The sensor system according to claim 1 wherein the product comprises a disposable absorbent article (300).

9. The sensor system according to claim 1 wherein the product comprises a package containing a consumable good (500).

10. A method of determining product information, the method including steps of:
providing at least one product (300, 400, 500) including a tag (100),
the tag including at least one sensor (110) adapted to transition from a first electrical state to a second electrical state in response to a predetermined change in an environment of the sensor (110),
the sensor having at least one output terminal;
a radio-frequency chip (130) including a memory element including electrical storage of a binary coded word including at least one bit, input terminals and output terminals,
the input terminals being disposed in electrical communication with the at least one output terminal of the at least one sensor (110) and with at least one bit of memory;
a first antenna (140) disposed in electrical communication with the output terminals of the chip (130);
providing, or having provided, an interrogator (200) adapted to detect radiation associated with an environmental state of the tag sensor (110);
interrogating the state of the tag (100);
interpreting the state of the tag (100);
providing an output associated with the interpreted state of the tag (100);
**characterized in that** the product comprises a battery, wherein the tag and sensor detect an open circuit voltage of the battery.

11. The method according to claim 10 wherein the step of providing an interrogator (200) further comprises providing an interrogator (200) including a network communications link (260), the method further including the step of sharing data associated with the state of the tag (100) over a network.

12. The method according to claim 11 further including the step of sharing data from the interrogator sensor (210) over the network.

13. The method according to claim 12 further including the step of purchasing a unit of the product (300, 400, 500) using the network.

## Patentansprüche

1. Sensorsystem, das ein Etikett (100) und ein Produkt (300, 400, 500) einschließt,
das Etikett (100) einschließend: wenigstens einen Sensor (110), der für den Übergang von einem ersten elektrischen Zustand in einen zweiten elektrischen Zustand als Reaktion auf eine vorbestimmte Änderung in einer Umgebung des Sensors (110) geeignet ist, wobei der Sensor (110) mindestens einen Ausgangsanschluss aufweist;
einen Hochfrequenzchip (130), der ein Speicherelement einschließlich elektrischer Speicherung eines binär codierten Wortes einschließend wenigstens ein Bit einschließt, Eingangsanschlüsse und Ausgangsanschlüsse, wobei die Eingangsanschlüsse in elektrischer Verbindung mit dem wenigstens einen Ausgangsanschluss des wenigstens einen Sensors (110) und mit wenigstens einem Bit des Speichers stehen;
eine erste Antenne (140), die in elektrischer Verbindung mit den Ausgangsanschlüssen des Chips steht;
**dadurch gekennzeichnet, dass** das Produkt eine Batterie umfasst, wobei das Etikett und der Sensor zum Erfassen einer Leerlaufspannung der Batterie konfiguriert sind.

2. Sensorsystem nach Anspruch 1, wobei der mindestens eine Ausgangsanschluss des Sensors (110) in elektrischer Verbindung mit mehreren Bits des Speichers steht.

3. Sensorsystem nach Anspruch 1, ferner einschließend eine Abfrageeinrichtung (200) einschließlich einer Stromquelle (220) und einer zweiten Antenne (230), die zum Erzeugen einer elektromagnetischen Strahlung einschließlich einer Resonanzfrequenz der ersten Antenne (140) geeignet ist, und einen Empfänger, der zum Erfassen elektromagnetischer Strahlung und zum Demodulieren der erfassten Strahlung durch Extrahieren eingebetteter Daten aus der erfassten Strahlung konzipiert ist.

4. System nach Anspruch 3, wobei die Abfrageeinrichtung (200) eine Netzwerkkommunikationsverbindung (260) einschließt.

5. Sensorsystem nach Anspruch 3, wobei die Abfrageeinrichtung (200) einen Sensor (210) umfasst.

6. Sensorsystem nach Anspruch 3, wobei die eingebetteten Daten den Wert des wenigstens einen Bits umfassen.

7. Sensorsystem nach Anspruch 1, ferner einschließend eine elektrische Abschirmung (150), die zwischen der Antenne und dem Produkt vorgesehen ist.

8. Sensorsystem nach Anspruch 1, wobei das Produkt einen absorbierenden Einwegartikel (300) umfasst.

9. Sensorsystem nach Anspruch 1, wobei das Produkt eine Verpackung umfasst, die ein Verbrauchsgut (500) enthält.

10. Verfahren zum Bestimmen von Produktinformationen, wobei das Verfahren folgende Schritte einschließt:
Bereitstellen mindestens eines Produkts (300, 400, 500) einschließlich eines Etiketts (100), wobei das Etikett wenigstens einen Sensor (110) einschließt, der für den Übergang von einem ersten elektrischen Zustand in einen zweiten elektrischen Zustand als Reaktion auf eine vorbestimmte Änderung in einer Umgebung des Sensors (110) geeignet ist, wobei der Sensor mindestens einen Ausgangsanschluss aufweist;
einen Hochfrequenzchip (130), der ein Speicherelement einschließlich elektrischer Speicherung eines binär codierten Wortes aufweisend wenigstens ein Bit umfasst, Eingangsanschlüsse und Ausgangsanschlüsse, wobei die Eingangsanschlüsse in elektrischer Verbindung mit dem wenigstens einen Ausgangsanschluss des wenigstens einen Sensors (110) und mit wenigstens einem Bit des Speichers stehen;
einer ersten Antenne (140), die in elektrischer Verbindung mit den Ausgangsanschlüssen des Chips (130) angeordnet ist;
Bereitstellen einer Abfrageeinrichtung (200), die zum Erfassen der mit einem Umgebungszustand des Sensors (110) verbundenen Strahlung konzipiert ist;
Abfragen des Zustands des Etiketts (100);
Interpretieren des Zustands des Etiketts (100);
Bereitstellen einer mit dem interpretierten Zustand des Etiketts (100) verbundenen Ausgabe;
**dadurch gekennzeichnet, dass** das Produkt eine Batterie umfasst, wobei das Etikett und der Sensor eine Leerlaufspannung der Batterie erfassen.

11. Verfahren nach Anspruch 10, wobei der Schritt des Bereitstellens einer Abfrageeinrichtung (200) ferner das Bereitstellen einer Abfrageeinrichtung (200) einschließlich einer Netzwerkkommunikationsverbindung (260) umfasst, wobei das Verfahren ferner den Schritt des Teilens der mit dem Zustand des Etiketts (100) verbundenen Daten über ein Netzwerk einschließt.

12. Verfahren nach Anspruch 11, ferner einschließend den Schritt des Teilens der Daten vom Sensor der Abfrageeinrichtung (210) über das Netzwerk.

13. Verfahren nach Anspruch 12, ferner einschließend den Schritt des Kaufens einer Einheit des Produkts (300, 400, 500) unter Verwendung des Netzwerks.

## Revendications

1. Système de capteur incluant une étiquette (100) et un produit (300, 400, 500),
l'étiquette (100) incluant : au moins un capteur (110) conçu pour passer d'un premier état électrique à un deuxième état électrique en réponse à un changement prédéterminé dans un environnement du capteur (110), le capteur (110) possédant au moins une borne de sortie ;
une puce à radiofréquence (130) incluant un élément de mémoire incluant un stockage électrique d'un mot à codage binaire incluant au moins un bit, des bornes d'entrée et des bornes de sortie, les bornes d'entrée étant disposées en communication électrique avec ladite au moins une borne de sortie dudit au moins un capteur (110) et avec au moins un bit de mémoire ;
une première antenne (140) étant disposée en communication électrique avec les bornes de sortie de la puce.
**caractérisé en ce que** le produit comprend une batterie, dans lequel l'étiquette et le capteur sont configurés pour détecter une tension de circuit ouvert de la batterie.

2. Système de capteur selon la revendication 1, dans lequel ladite au moins une borne de sortie du capteur (110) est disposée en communication électrique avec plusieurs bits de la mémoire.

3. Système de capteur selon la revendication 1, incluant en outre un interrogateur (200) incluant une source d'alimentation (220) et une deuxième antenne (230) conçue pour générer un rayonnement électromagnétique incluant une fréquence de résonance de la première antenne (140), et un récepteur conçu pour détecter un rayonnement électromagnétique et démoduler le rayonnement détecté en extrayant les données intégrées du rayonnement détecté.

4. Système selon la revendication 3, dans lequel l'interrogateur (200) inclut une liaison de communications réseau (260).

5. Système de capteur selon la revendication 3, dans lequel l'interrogateur (200) comprend un capteur (210).

6. Système de capteur selon la revendication 3, dans lequel les données intégrées comprennent la valeur dudit au moins un bit.

7. Système de capteur selon la revendication 1, incluant en outre un blindage électrique (150) disposé entre l'antenne et le produit.

8. Système de capteur selon la revendication 1, dans lequel le produit comprend un article absorbant jetable (300).

9. Système de capteur selon la revendication 1, dans lequel le produit comprend un emballage contenant un article consommable (500).

10. Procédé de détermination d'informations de produit, le procédé incluant les étapes consistant à :
fournir au moins un produit (300, 400, 500) incluant une étiquette (100), l'étiquette incluant au moins un capteur (110) conçu pour passer d'un premier état électrique à un deuxième état électrique en réponse à un changement prédéterminé dans un environnement du capteur (110), le capteur possédant au moins une borne de sortie ;
une puce à radiofréquence (130) incluant un élément de mémoire incluant un stockage électrique d'un mot à codage binaire incluant au moins un bit, des bornes d'entrée et des bornes de sortie, les bornes d'entrée étant disposées en communication électrique avec ladite au moins une borne de sortie dudit au moins un capteur (110) et avec au moins un bit de mémoire ;
une première antenne (140) disposée en communication électrique avec les bornes de sortie de la puce (130) ;
fournir, ou avoir fourni, un interrogateur (200) conçu pour détecter un rayonnement associé à un état environnemental du capteur d'étiquette (110) ;
interroger l'état de l'étiquette (100) ;
interpréter l'état de l'étiquette (100) ;
fournir une sortie associée à l'état interprété de l'étiquette (100),
**caractérisé en ce que** le produit comprend une batterie, dans lequel l'étiquette et le capteur détectent une tension de circuit ouvert de la batterie.

11. Procédé selon la revendication 10, dans lequel l'étape de fourniture d'un interrogateur (200) comprend en outre la fourniture d'un interrogateur (200) incluant une liaison de communications réseau (260), le procédé incluant en outre l'étape consistant à partager les données associées à l'état de l'étiquette (100) sur un réseau.

12. Procédé selon la revendication 11, incluant en outre l'étape consistant à partager les données provenant du capteur d'interrogateur (210) sur le réseau.

13. Procédé selon la revendication 12, incluant en outre l'étape consistant à acheter une unité du produit (300, 400, 500) en utilisant le réseau.
